# EUROPÄISCHE PATENTANMELDUNG

(11) **EP 1 450 086 A1**
(43) Veröffentlichungstag der Anmeldung: **25.08.2004**
(21) Anmeldenummer: 04090057.3
(22) Anmeldetag: 18.02.2004
(51) Int. Cl.: F16K 17/08

(54) **Dichtelement**

(30) Priorität: 20.02.2003 DE 10308163; 13.05.2003 DE 10322244
(71) Anmelder: Biotronik GmbH & Co. KG, 12359 Berlin (DE)
(72) Erfinder: Agur, Junge, 10787 Berlin (DE)
(74) Vertreter: Eisenführ, Speiser & Partner

(57) **Zusammenfassung**

Die Erfindung betrifft ein Dichtelement (10) aus elastischem Material mit einem rohrförmigen Grundkörper (12), dessen Umfangswandung (22) einen Hohlraum mit einer Durchtrittsöffnung (24) für Fluide einschließt, wobei die Umfangswandung (22) im Bereich der Durchtrittsöffnung (24) in Bezug auf Elastizität des Materials, Wandstärke und Innendurchmesser derart ausgebildet ist, dass eine Torsion des Grundkörpers (12) eine Einschnürung des Hohlraums im Bereich der Durchtrittsöffnung (24) bewirkt.

## Beschreibung

Die Erfindung betrifft ein Dichtelement aus elastischem Material mit einem rohrförmigen Grundkörper und zwei Längsenden, dessen Umfangswandung einen Hohlraum mit einer Durchtrittsöffnung für Fluide einschließt.

Solche Dichtelemente finden beispielsweise in Form von Stopfbuchsen als hämostatische Ventile bei Einführkathetern Verwendung. Einführkatheter werden beispielsweise im Zusammenhang mit dem Implantieren von Elektrodenleitungen für Herzschrittmacher oder Defibrillatoren verwendet. Ein Einführkatheter umfasst üblicherweise eine längliche flexible Röhre, durch die hindurch beispielsweise die Elektrodenleitung in ein Blutgefäß eingeführt werden kann. Zum Einführen der Elektrodenleitung muss der Einführkatheter sowohl am distalen als auch am proximalen Ende für die Elektrodenleitung offen sein. Gleichzeitig ist möglichst weitgehend zu verhindern, dass durch den notwendigerweise an beiden Enden offenen Einführkatheter Blut austritt. Zu diesem Zweck ist üblicherweise im Bereich der dem Einführen der Elektrodenleitung dienenden Einführöffnung am proximalen Ende des Einführkatheters ein hämostatisches Ventil vorgesehen, welches aus elastischem Material besteht und die Elektrodenleitung eng genug umschließt, um einen Austritt von Blut zu verhindern.

Solche hämostatischen Ventile sind üblicherweise als Stopfbuchsen oder als geschlitzte Dichtscheiben ausgeführt.

Aus der US 5,464,189 ist außerdem ein Katheterventil mit einer variablen Öffnung bekannt, welches sich durch Torsion einschnüren lässt, so dass sich eine Durchtrittsöffnung mit variablem Durchmesser ergibt.

Die Handhabung derartiger hämostatischer Ventile lässt in der Regel zu wünschen übrig. Insbesondere in Bezug auf die US 5,464,189 ist es von Nachteil, dass das dort vorgestellte Dichtelement sich über die gesamte Länge des nicht eingespannten Abschnittes (freie Länge) des Dichtelementes erstreckt. Um bei einem derartigen Dichtelement eine gewünschte, sich membranartig schließende Durchtrittsöffnung zu erzielen, muss die freie Länge des Dichtelementes im Verhältnis zum Durchmesser sehr kurz gewählt werden, so wie dies in Figur 30 oder 34 der US 5,464,189 dargestellt ist. Andernfalls ergibt sich eine für das Einführen von beispielsweise Elektroden durch die Durchtrittsöffnung ungünstige Form der Einschnürung, wie sie in den Figuren 4,7,10 oder 13 der US 5,464,189 abgebildet ist.

Der Erfindung liegt vor diesem Hintergrund die Aufgabe zu Grunde, ein hämostatisches Ventil anzugeben, welches Nachteile bekannter hämostatischer Ventile hinsichtlich deren Handhabung oder deren universeller Verwendbarkeit möglichst vermeidet.

Erfindungsgemäß besteht die Lösung dieser Aufgabe in einem Dichtelement der Eingangs genannten Art, dessen Umfangswandung im Bereich der Durchtrittsöffnung im Bezug auf Elastizität des Materials, Wandstärke und Innendurchmesser derart ausgebildet ist, dass eine Torsion der beiden Längsenden relativ zueinander eine regelmäßige Faltung der Umfangswandung im Bereich der Durchtrittsöffnung und damit einhergehend eine von einem Torsionswinkel abhängige Verringerung des Durchmessers der Durchtrittsöffnung derart bewirkt, dass sich die Einschnürung in Bezug auf die Längsrichtung des Dichtelementes an vorgegebener Position befindet.

Der Erfindung liegt die Erkenntnis zu Grunde, dass es bei geeigneter, auf die Materialeigenschaften angepasster Bemaßung eines rohrförmigen Grundkörper aus elastischem Material zu einer Einschnürung des Grundkörpers kommt, wenn der Grundkörper bezüglich der Längsachse seiner Durchtrittsöffnung tordiert wird. Im verallgemeinerten Sinne bezieht sich die Erfindung somit auf ein Dichtelement aus elastischem Material mit einem rohrförmigen Grundkörper, dessen Durchtrittsöffnung durch Torsion des Grundkörpers bezüglich einer Längsachse der Durchtrittsöffnung einzuschnüren ist.

Dieses Prinzip der Einschnürung eines rohrförmigen Grundkörpers aus elastischem Material durch Torsion ermöglicht es, den Durchmesser der Durchtrittsöffnung an den jeweiligen Anwendungsfall durch Wahl des Torsionswinkels anzupassen. Es hat sich gezeigt, dass sich mit einem derartigen Dichtelement erstaunlich reproduzierbar sehr gute Dichtwirkungen erzielen lassen.

Ein Dichtkörper mit einer im Verhältnis zum Durchmesser der Durchtrittsöffnung geringen Wandstärke aus einem besonders biegeweichen Material wie beispielsweise Silikonkautschuk zeigt die hier gewünschten Eigenschaften.

In einer bevorzugten Ausführungsvariante ist die Wandstärke der Umfangswandung im Bereich der Durchtrittsöffnung geringer, als in weiter von der Durchtrittsöffnung entfernten Bereichen des Dichtelementes. Besonders bevorzugt ist es, wenn die Wandstärke mit zunehmendem Abstand von der Durchtrittsöffnung größer wird oder nur im Breich der Durchtrittsöffnung vermindert ist. Im Bereich der Durchtrittsöffnung ist eine Wandstärke von Vorteil, die so gering wie möglich ist, damit eine ausreichende Dichtigkeit und Festigkeit des Dichtelementes gewahrt bleibt. Dabei ist es vorteilhaft, wenn die Verminderung der Wandstärke durch eine Verminderung des Außendurchmessers des Dichtelementes bewirkt ist, so dass der Hohlraum im Inneren des Dichtelementes in seinem entspannten, vollständig geöffneten Zustand die Form eines an seinen Stirnseiten offenen Zylinders hat.

Vorzugsweise besitzt das Dichtelement an seinen beiden Längsenden jeweils einen sich radial nach außen erstreckenden Flansch. Mit diesen Flanschen können die Längsenden des Dichtelementes leicht mit Stellelementen für die Torsion des Dichtelementes verbunden werden.

Weiterhin weist das Dichtelement in einer speziellen Ausführungsform wenigstens an einem Längsende eine Membran auf, die das Dichtelement zu mindest teilweise verschließt. Diese Membran weist vorzugsweise eine zentrale Öffnung auf, deren Durchmesser kleiner ist als der Durchmesser der Durchtrittsöffnung im voll geöffneten Zustand. Die Membran wirkt beispielsweise beim Einführen einer Elektrodenleitung als ein zweites, jedoch nicht verstellbares hämostatisches Ventil.

Geeignete Materialien für ein derartiges Dichtelement sind grundsätzlich Kautschuke, insbesondere Silikonkautschuk mit einer Härte größer 30 Shore und vorzugsweise zwischen 50 und 70 Shore. Das Dichtelement ist dabei vorzugsweise einstückig ausgeführt und besteht aus einem homogenen Werkstoff.

Ein weiterer Erfindungsaspekt betrifft einen Einführkatheter mit einem Dichtelement, welches beispielsweise als hämostatisches Ventil wirkt. Bekannte Katheter lassen bezüglich der Einstellbarkeit und der Handhabung der verwendeten Dichtelemente einiges zu wünschen übrig. Dementsprechend liegt der vorliegenden Erfindung weiterhin die Aufgabe zu Grunde, auch einen Einführkatheter mit verbessertem Dichtelement anzubieten.

Erfindungsgemäß wird diese Aufgabe durch einen Einführkatheter mit einem Dichtelement der hier beanspruchten Art gelöst.

Das Dichtelement ist vorzugsweise im Bereich einer Einführöffnung des Einführkatheters angeordnet. Eine derartige Einführöffnung dient dem Einführen beispielsweise von Elektrodenleitungen, Führungsdrähten oder auch Kathetern zum Positionieren von intravaskulären Gefäßprothesen.

Das Dichtelement ist dabei vorzugsweise zum wahlweisen Verschließen oder Öffnen der Einführöffnung angeordnet und ausgebildet. Dazu weist der Einführkatheter vorzugsweise zwei relativ zueinander verdrehbare Stellelemente auf, die mit jeweils einem Längsende des Dichtelementes zum Einstellen des Durchmessers der Durchtrittsöffnung des Dichtelementes wirkverbunden sind. Diese Stellelemente sind vorzugsweise im Bereich der Einführöffnung des Einführkatheters angeordnet und somit für einen Arzt gut zu erreichen. Gleichzeitig ist eine unmittelbare Nähe zu dem zu verstellenden Dichtelement möglich.

Die Stellelemente sind vorzugsweise derart ausgeführt, dass sie verschiedene relativ zueinander verdrehte Positionen nach dem Einstellen beibehalten. Auf diese Weise bleibt ein einmalig eingestellter Torsionswinkel und der damit vorgegebene Durchmesser der Durchtrittsöffnung nach dem Einstellen erhalten. Hierzu kann es in einer bevorzugten Ausführungsvariante vorgesehen sein, die Stellelemente so auszubilden, dass sie in verschiedenen, relativ zueinander verdrehten Positionen einrasten.

Die Erfindung soll nun anhand eines Ausführungsbeispiels mit Hilfe der beigefügten Figuren näher erläutert werden.

Von den Figuren zeigen:
- Figur 1: Eine Seitenansicht eines erfindungsgemäßen Dichtelementes;
- Figur 2: Das Dichtelement aus Figur 1 in tordierten Zustand;
- Figur 2a: eine erste Variante der Faltung des Dichtelementes;
- Figur 2b: eine zweite Variante der Faltung des Dichtelementes;
- Figur 3: eine alternaive Ausführungsvariante des Dichtelementes in geschnittener Darstellung;
- Figur 4a: ein erfindungsgemäßes hämostatisches Ventil in einer Seitenansicht;
- Figur 4b: ein Längsschnitt durch das hämostatische Ventil aus Figur 4a;
- Figur 5: Elemente eines distalen Endes eines Einführkatheters mit einem hämostatischen Ventil gemäß Figuren 4a und 4b in geschnittener Darstellung; und
- Figur 6: eine weitere, zu Figur 3 alternative Ausführungsvariante des Dichtelementes in geschnittener Darstellung.

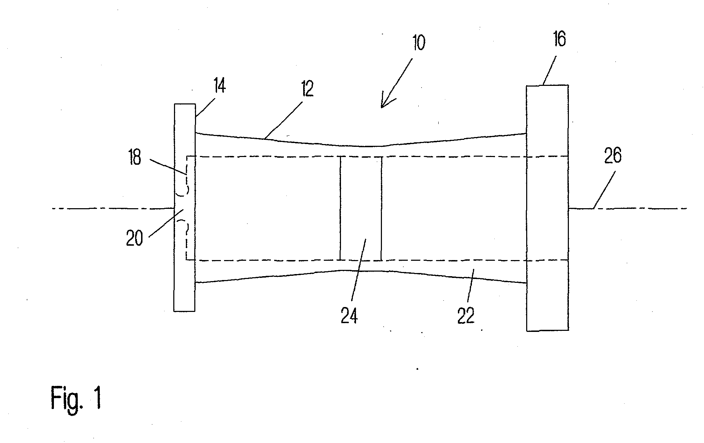

Das in Figur 1 dargestellte Dichtelement 10 weist einen rohrförmigen Grundkörper 12 auf, an dessen Längsenden sich radial nach außen erstreckende Flansche 14 und 16 anschließen. Das Dichtelement 10 besteht aus weichem oder mittelhartem Silikonkautschuk mit einer Shore-Härte von 30 oder mehr. Im Bereich des Flansches 14 ist eine sich nach Innen erstreckende Membran 18 vorgesehen, die eine zentrale kreisrunde Öffnung 20 besitzt.

Der rohrförmige Grundkörper besitzt eine Umfangswandung 22, die eine zentrale Durchtrittsöffnung 24 des rohrförmigen Grundkörpers 12 umschließt. Der Durchmesser der Umfangswandung nimmt ausgehend vom Bereich der Durchtrittsöffnung 24 hin zu den Flanschen 14 und 16 stetig zu. Die Umfangswandung 22 besitzt somit im Bereich der Durchtrittsöffnung 24 die geringste Wandstärke. Der Durchmesser der Durchtrittsöffnung 24 beträgt beispielsweise 5 bis 6 mm, die Wandstärke der Umfangswandung 22 im Bereich der Durchtrittsöffnung 24 0,1 bis 0,3 mm.

Die Membran 18 hat ebenfalls eine Wandstärke von etwa 0,1 bis 0,3 mm. Die Öffnung 20 hat einen Durchmesser von 1 bis 2 mm.

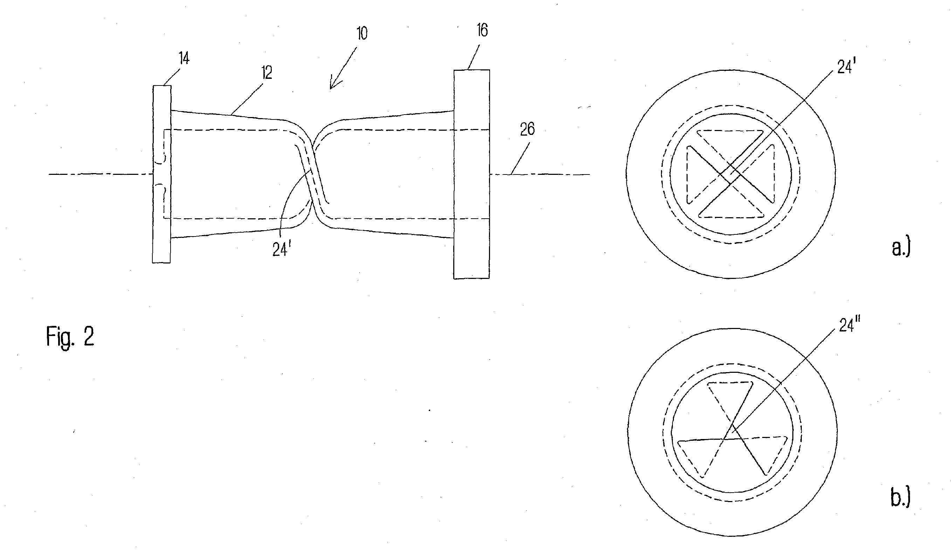

Figur 2 zeigt das Dichtelement 10 aus Figur 1 im tordierten Zustand. Dies ist der Zustand, in dem die beiden Flansche 14 und 16 um eine Längsachse 26 herum relativ zueinander verdreht sind. Es ist zu erkennen, dass sich im Bereich der Durchtrittsöffnung 24 eine Einschnürung bildet. Bei entsprechender Gestaltung der Umfangswandung 22 ist diese Einschnürung regelmäßig, indem sich die Umfangswandung 22 im Bereich der Durchtrittsöffnung 24 wie in den Figuren 2a und 2b dargestellt regelmäßig faltet. Die Durchtrittsöffnung 24 (Figur 2a) bzw. 24' (Figur 2b) bildet sich auf diese Weise irisförmig aus und weist eine außerordentlich hohe Dichtigkeit auf. Die eingeschnürten Durchtrittsöffnungen 24' und 24" haben offensichtlich einen wesentlich kleineren Durchmesser als die voll geöffnete Durchtrittsöffnung 24 aus Figur 1.

Entscheidend ist, dass sich der Durchmesser der irisförmigen Durchtrittsöffnungen 24' und 24" durch den Winkelgrad einstellen lässt, um den die beiden Flansche 14 und 16 gegeneinander verdreht sind. Eine geringere Verdrehung der beiden Flansche 14 und 16 zueinander führt zu einer kleineren Verengung der Durchtrittsöffnung 24 also zu einem größeren Durchmesser der irisförmigen Durchtrittsöffnungen 24' bzw. 24". Zu beachten ist, dass es für die wesentliche Eigenschaft des Dichtelementes 10 nicht auf die Flansche 14 und 16 selbst ankommt. Auch ohne diese Flansche hat das Dichtelement 10 die beschriebenen Eigenschaften. Die Flansche 14 und 16 haben im wesentlichen den Zweck, eine Anschlussmöglichkeit für Stellelemente zum Verstellen des Dichtelementes 10 zu bieten.

Die Membran 18 mit ihrer zentralen Öffnung 20 dient als zweites, hilfsweises Dichtelement im Falle des Einführens einer Elektrodenleitung.

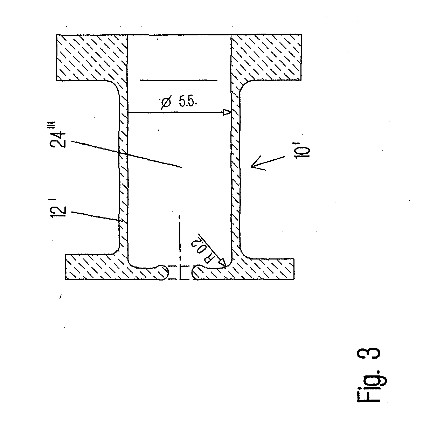

In Figur 3 ist eine alternative Ausführungsvariante eines Dichtelementes 10' in geschnittener Darstellung abgebildet. Die Maße für die Wandstärke der Umfangswandung 22' und der Durchtrittsöffnung 24"' entsprechen in etwa denen der Ausführungsvariante aus Figur 1.

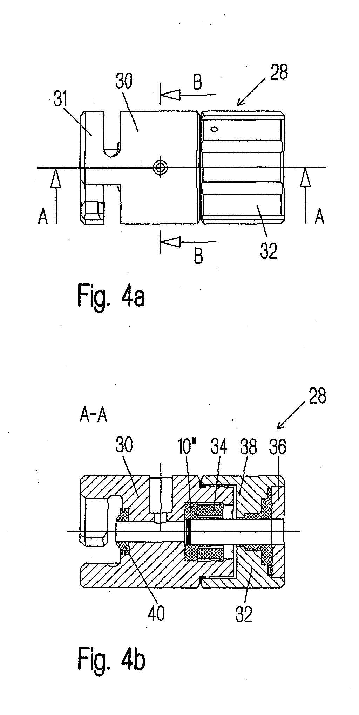

Die Figuren 4a und 4b zeigen ein hämostatisches Ventil 28 wie es als wesentliches Anschlusselement für einen in den Figuren 4a und 4b nicht dargestellten Einführkatheter zu verwenden ist. Das hämostatische Ventil 28 umfasst ein distales Katheteranschlusselement 30 und ein Stellelement, welche rastend miteinander verbunden sind und relativ zueinander verdrehbar ausgeführt sind. Das Katheteranschlusselement 30 weist einen Bajonett-Anschluss 31 auf, mit dem das als Gehäuse des hämostatischen Ventils dienende Katheteranschlusselement 30 mit einem Katheter zu verbinden ist. Das Stellelement 32 ist als Drehring ausgebildet. Das distale Anschlusselement 30 und das Stellelement 32 bilden ein erstes und ein zweites Stellelement im Sinne der Beschreibungseinleitung.

Das distale Anschlusselement 30 und das Stellelement 32 schließen ein Dichtelement 10" der vorbeschriebenen Art ein. Dabei ist jeweils das distale Anschlusselement 30 und das Stellelement 32 mit jeweils einem Flansch des Dichtelementes 10" verbunden. Mit Hilfe eine Klemmbuchse 34 und einem Klemmstift 38 ist ein Rastmechanismus verwirklicht, der bewirkt, dass das distale Anschlusselement 30 und das Stellelement 32 in verschiedenen relativ zueinander verdrehten Zuständen einrasten. Auf diese Weise lässt sich eine definierte Einschnürung des Dichtelementes 10" durch Verdrehen des Stellelementes 32 gegenüber dem distalen Anschlusselement 30 bewirken. Diese Einstellung bleibt auch nach Loslassen des Stellelementes 32 erhalten. Durch relatives Verdrehen des Stellelementes 32 bezüglich des distalen Anschlusselementes 30 werden die beiden Flansche des Dichtelementes 10" relativ zueinander verdreht, mit der Folge, dass sich das Dichtelement 10" wie in Figur 2 abgebildet einschnürt. Der distale Flansch des Stellelementes 32 ist zu diesem Zweck mit Hilfe einer Klemmplatte 36 fest mit dem Stellelement 32 verbunden.

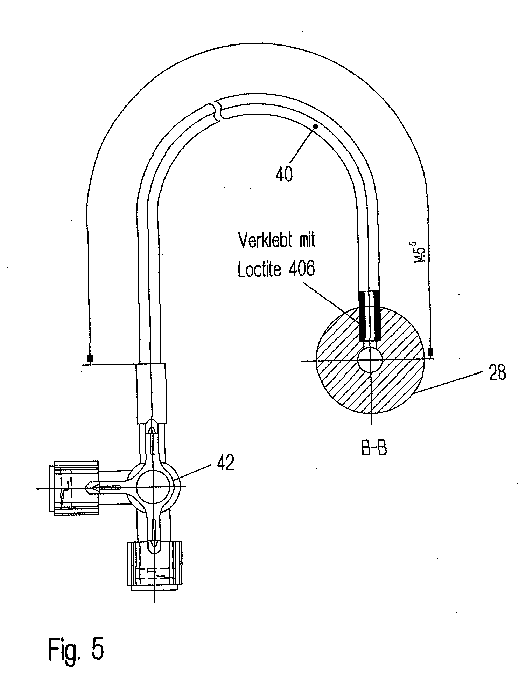

In Figur 5 ist außerdem ein Schlauch 40 mit daran angeschlossenem 3-Wege-Hahn 42 dargestellt. Außerdem ist das hämostatische Ventil 28 in einem Querschnitt B-B dargestellt.

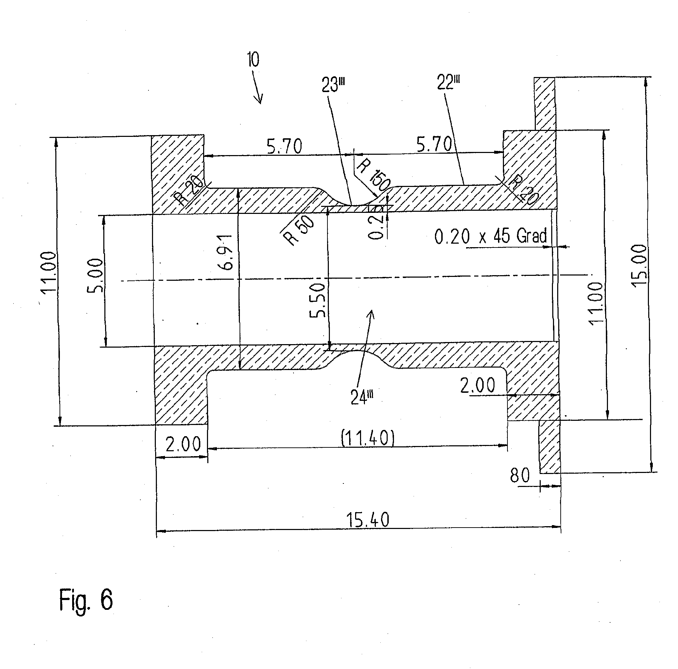

In Figur 6 ist eine weitere alternative Ausführungsform eines Dichtelementes 10'" ähnlich Figur 1 oder Figur 3 dargestellt. Das in Figur 6 dargestellte Dichtelement 10"' besteht aus Silikonkautschuk mit einer Shore-Härte zwischen 50 und 70.

Die Maße des Dichtelemenetes 10'" können dem Längsschnitt in Figur 6 entnommen werden. Die Maße sind in Millimetern angegeben.

Zu erkennen ist, dass bei dem Dichtelement 10"' gemäß Figur 6 die Verminderung der Wandstärke der Umfangswandung 22"' im Bereich der Durchtrittsöffnung 24"' durch eine Ringnut 23"' auf der Außenseite der Umfangswandung 22"' bewirkt ist. Durch die Ringnut 23"' wird bewirkt, dass sich das Dichtelement 10"' beim Tordieren nur im Bereich der Ringnut 23"' und in deren Nähe einschnürt. Damit ist auch der Ort der Durchtrittsöffnung 24"' in Bezug auf die Längsrichtung des Dichtelementes 10"' festgelegt.

## Patentansprüche

1. Dichtelement (10) aus elastischem Material mit einem rohrförmigen Grundkörper (12), dessen Umfangswandung (22) einen sich entlang einer Längsrichtung des Dichtelementes erstreckenden Hohlraum mit einer Durchtrittsöffnung (24) für Fluide einschließt, wobei die Umfangswandung (22) im Bereich der Durchtrittsöffnung (24) in Bezug auf Elastizität des Materials, Wandstärke und Innendurchmesser derart ausgebildet ist, dass eine Torsion des Grundkörpers (12) eine Einschnürung des Hohlraums im Bereich der Durchtrittsöffnung (24) derart bewirkt, dass sich die Einschnürung in Bezug auf die Längsrichtung des Dichtelementes an vorgegebener Position befindet.

2. Dichtelement (10) nach Anspruch 1 mit zwei Längsenden, **dadurch gekennzeichnet, dass** die Umfangswandung (22) im Bereich der Durchtrittsöffnung (24) in Bezug auf Elastizität des Materials, Wandstärke und Innendurchmesser derart ausgebildet ist, dass eine Torsion der beiden Längsenden relativ zueinander eine regelmäßige Faltung der Umfangswandung (22) im Bereich der Durchtrittsöffnung (24) und damit einhergehend eine vom einem Torsionswinkel abhängige Verringerung des Durchmessers der Durchtrittsöffnung (24) bewirkt.

3. Dichtelement (10) nach Anspruch 1 oder 2, **dadurch gekennzeichnet, dass** die Umfangswandung (22) im Bereich der Durchtrittsöffnung (24) eine geringere Wandstärke aufweist als in benachbarten Wandbereichen.

4. Dichtelement (10) nach Anspruch 3, **dadurch gekennzeichnet, dass** die Wandstärke der Umfangswandung (22) mit zunehmendem Abstand von der Durchtrittsöffnung (24) stetig zunimmt.

5. Dichtelement (10) nach Anspruch 3, **dadurch gekennzeichnet, dass** die Wandstärke der Umfangswandung (22) nur im Bereich der Durchtrittsöffnung vermindert und im übrigen im wesentlichen konstant ist.

6. Dichtelement (10) nach Anspruch 2, **gekennzeichnet durch** einen Flansch (14, 16) an einem Längsende des Dichtelementes (10), der sich bezüglich des rohrförmigen Grundkörpers (12) radial nach außen erstreckt.

7. Dichtelement (10) nach Anspruch 6, **gekennzeichnet durch** jeweils einen Flansch (14, 16) an den Längsenden des Dichtelementes (10).

8. Dichtelement (10) nach Anspruch 2, **gekennzeichnet durch** eine Membran (18), die wenigstens ein Längsende des Dichtelementes (10) zumindest teilweise verschließt.

9. Dichtelement (10) nach Anspruch 8, **dadurch gekennzeichnet, dass** die Membran (18) eine zentrale Öffnung (20) aufweist, deren Durchmesser kleiner ist, als der Durchmesser der Durchtrittsöffnung (24) im voll geöffneten Zustand.

10. Dichtelement (10) nach Anspruch 1, **dadurch gekennzeichnet, dass** das Dichtelement (10) aus einem Silikonkautschuk enthaltenden Material besteht.

11. Dichtelement (10) nach Anspruch 1 oder 10, **dadurch gekennzeichnet, dass** das Dichtelement (10) aus einem Material mit einer Shore-Härte größer 30 besteht.

12. Dichtelement (10) nach Anspruch 11, **dadurch gekennzeichnet, dass** das Dichtelement (10) aus einem Material mit einer Shore-Härte größer 50 besteht.

13. Dichtelement (10) nach Anspruch 12, **dadurch gekennzeichnet, dass** das Dichtelement (10) aus einem Material mit einer Shore-Härte zwischen 50 und 70 besteht.

14. Dichtelement (10) nach Anspruch 1, **dadurch gekennzeichnet, dass** der Hohlraum bei vollständig geöffneter Durchtrittsöffnung einen über die Längsrichtung im wesentlichen einheitlichen runden Durchmesser hat, so dass der Hohlraum die Form eines an seinen beiden Stirnseiten offenen Zylinders hat.

15. Einführkatheter mit einem Dichtelement (10") nach Anspruch 1.

16. Einführkatheter nach Anspruch 15, **dadurch gekennzeichnet, dass** das Dichtelement im Bereich einer Einführöffnung für mit Hilfe des Einführkatheters einzuführende Elektrodenleitungen, Führungsdrähte oder dergleichen angeordnet ist.

17. Einführkatheter nach Anspruch 16, **dadurch gekennzeichnet, dass** das Dichtelement (10") zum wahlweisen Verschließen oder Öffnen der Einführöffnung angeordnet und ausgebildet ist.

18. Einführkatheter nach Anspruch 17, **dadurch gekennzeichnet, dass** der Einführkatheter zwei relativ zueinander verdrehbare Stellelemente (30, 32) aufweist, die mit jeweils einem Längsende des Dichtelementes (10") zum Einstellen des Durchmessers der Durchtrittsöffnung des Dichtelementes wirkverbunden sind.

19. Einführkatheter nach Anspruch 18, **dadurch gekennzeichnet, dass** die Stellelemente (30, 32) im Bereich der Einführöffnung des Einführkatheters angeordnet sind.

20. Einführkatheter nach Anspruch 18 oder 19, **dadurch gekennzeichnet, dass** die Stellelemente (30, 32) verschiedene relativ zueinander verdrehte Positionen nach dem Einstellen beibehalten.

21. Einführkatheter nach Anspruch 20, **dadurch gekennzeichnet, dass** die Stellelemente (30, 32) in verschiedenen relativ zueinander verdrehten Positionen einrasten.
